# EUROPÄISCHE PATENTSCHRIFT

(11) **EP 1 139 739 B1**
(45) Veröffentlichungstag und Bekanntmachung des Hinweises auf die Patenterteilung: **11.09.2002**
(21) Anmeldenummer: 99958168.9
(22) Anmeldetag: 06.12.1999
(51) Int. Cl.: A01N 25/30, A01N 43/653, A01N 43/88

(54) **AGROCHEMISCHE FORMULIERUNGEN**
AGROCHEMICAL FORMULATIONS
COMPOSITIONS AGROCHIMIQUES

(30) Priorität: 16.12.1998 DE 19857963
(43) Veröffentlichungstag der Anmeldung: 10.10.2001
(73) Patentinhaber: Bayer Aktiengesellschaft, 51368 Leverkusen (DE)
(72) Erfinder: RÖCHLING, Andreas, D-40764 Langenfeld (DE); SUTY-HEINZE, Anne, D-40764 Langenfeld (DE); REIZLEIN, Karl, D-51061 Köln (DE); RECKMANN, Udo, D-50823 Köln (DE)
(86) Internationale Anmeldenummer: EP9909528
(87) Internationale Veröffentlichungsnummer: WO00035278

(56) Entgegenhaltungen:
- EP-A- 0 355 759
- EP-A- 0 681 865
- WO-A-97/04653
- WO-A-98/35553
- GB-A- 2 269 102

## Beschreibung

Die vorliegende Erfindung betrifft neue agrochemische Formulierungen auf Basis bestimmter 2-Ethyl-hexanol-alkoxylate, ein Verfahren zur Herstellung dieser Formulierungen und deren Verwendung zur Applikation der enthaltenen agrochemischen Wirkstoffe.

Es sind bereits zahlreiche Formulierungen von Pflanzenbehandlungsmitteln bekannt, die Fettalkohol-ethoxylate als Netzmittel bzw. Penetrationsförderer enthalten (vgl. EP-A 0 579 052). Die Wirksamkeit dieser Zubereitungen ist gut. Nachteilig ist jedoch, daß beim Verrühren mit Wasser in manchen Fällen eine starke Schaumbildung eintritt.

Weiterhin wurden schon Formulierungen von Agrochemikalien beschrieben, in denen Fettalkohol-propoxylate als Formulierhilfsmittel vorhanden sind (vgl. US-A 3 673 087). Auch die Eigenschaften dieser Zubereitungen sind aber nicht immer ausreichend, da Propoxylate mit längerem Alkylteil in Wasser wenig löslich sind und deshalb zur Abscheidung neigen.

Bekannt ist außerdem, daß sich Gemische aus Fettalkohol-ethoxylaten und -propoxylaten sowie deren Copolymere als schaumarme Netzmittel zur Formulierung von Wirkstoffen im Pflanzenschutz einsetzen lassen (vgl. EP-A 0 681 865). In der Praxis lassen aber auch die Eigenschaften derartiger Zubereitungen in manchen Fällen zu wünschen übrig.

Es wurden nun neue agrochemische Formulierungen gefunden, die aus
a) mindestens einem agrochemischen Wirkstoff,
b) 2-Ethyl-hexanol-alkoxylat der Formel 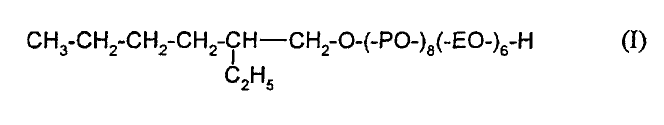 worin
   - P: für 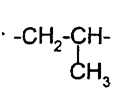 steht,
   - E: für -CH₂-CH₂- steht und
   die Zahlen 8 und 6 Durchschnittswerte darstellen,
   und
c) gegebenenfalls Zusatzstoffen
bestehen.

Weiterhin wurde gefunden, daß sich die erfindungsgemäßen agrochemischen Formulierungen herstellen lassen, indem man
- mindestens einen agrochemischen Wirkstoff
- mit 2-Ethyl-hexanol-alkoxylat der Formel (I) sowie
- gegebenenfalls mit Zusatzstoffen
vermischt.

Schließlich wurde gefunden, daß sich die erfindungsgemäßen agrochemischen Formulierungen sehr gut zur Applikation der enthaltenen Wirkstoffe auf Pflanzen und/oder deren Lebensraum eignen.

Es ist als äußerst überraschend zu bezeichnen, daß die erfindungsgemäßen Formulierungen die in ihrer Zusammensetzung ähnlichsten vorbekannten Zubereitungen hinsichtlich ihrer Eigenschaften deutlich übertreffen. Im übrigen war aufgrund der technischen Lehre, die aus der EP-A 0 681 865 zu entnehmen ist, davon auszugehen, daß ein Gemisch verschiedener Alkoxylate benötigt wird, damit die resultierenden Mittel allen Anforderungen der Praxis genügen. Im Gegensatz zu den Erwartungen ist das aber nicht der Fall. Speziell das Vorhandensein von 2-Ethyl-hexanol-alkoxylat der Formel (I) reicht aus, um Formulierungen mit dem gewünschten Eigenschaftsprofil zu erzeugen.

Die erfindungsgemäßen Formulierungen zeichnen sich durch eine Reihe von Vorteilen aus. So tritt beim Vermischen der erfindungsgemäßen Formulierungen mit Wasser nur eine sehr geringe Schaumbildung auf. Weiterhin begünstigen die Formulierungen die biologische Wirksamkeit der enthaltenen aktiven Komponenten. Vorteilhaft ist außerdem, daß in Wasser nur wenig lösliche Wirkstoffe in den erfindungsgemäßen Formulierungen und bei deren Verdünnen mit Wasser eine verminderte Neigung zur Kristallisation zeigen.

Die erfindungsgemäßen Formulierungen enthalten einen oder mehrere agrochemische Wirkstoffe. Unter agrochemischen Wirkstoffen sind hierbei alle zur Pflanzenbehandlung üblichen Substanzen zu verstehen. Vorzugsweise genannt seien Fungizide, Bakterizide, Insektizide, Akarizide, Nematizide, Herbizide, Pflanzenwuchsregulatoren, Pflanzennährstoffe und Repellents.

### Als Beispiele für Fungizide seien genannt:

2-Aminobutan; 2-Anilino-4-methyl-6-cyclopropyl-pyrimidin; 2',6'-Dibromo-2-methyl-4'-trifluoromethoxy-4'-trifluoromethyl-1,3-thiazol-5-carboxanilid; 2,6-Dichloro-N-(4-trifluoromethylbenzyl)-benzamid; (E)-2-Methoximino-N-methyl-2-(2-phenoxyphenyl) acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino[alpha-(otolyloxy)-o-tolyl]-acetat; 2-Phenylphenol (OPP),
Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Dichlofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Mancozeb, Maneb, Mepanipyrim, Mepronil, Metalaxyl, Metconazol,
Methasulfocarb, Methfuroxam, Metiram, Metsulfovax, Myclobutanil,
Nickeldimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),Quinoxyfen,
Tebuconazol, Tecloftalam, Tecazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram,
8-tert.-Butyl-2-(N-ethyl-N-n-propyl-amino)-methyl-1,4-dioxa-spiro-[4,5]decan,
N-(R)-[1-(4-Chlorphenyl)-ethyl]-2,2-dichlor-1-ethyl-3t-methyl-1r-cyclopropancarbonsäureamid (Diastereomerengemisch oder einzelne Isomere),
[2-Methyl-1-[[[1-(4-methylphenyl)-ethyl]-amino]-carbonyl]-propyl]-carbaminsäure-1-methylethylester,
1-Methyl-cyclohexyl-1-carbonsäure-(2,3-dichlor-4-hydroxy)-anilid,
2-[2-(1-Chlor-cyclopropyl)-3-(2-chlorphenyl)-2-hydroxypropyl]-2,4-dihydro-[1,2,4]-triazol-3-thion,
1-(3,5-Dimethyl-isoxazol-4-sulfonyl)-2-chlor-6,6-difluor-[1,3]-dioxolo-[4,5-f]-benzimidazol und
(5,6-Dihydro-1,4,2-dioxazin-3-yl)-{2-[[6-(2-chlor-phenoxy)-5-fluor-4-pyrimidinyl]-oxy]phenyl}-methanon-O-methyloxim.

Als Beispiele für Bakterizide seien genannt:
Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

Als Beispiele für Insektizide, Akarizide und Nematizide seien genannt:
Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-lH-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chloretoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton-M, Demeton-S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin, Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram,
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimiphos, Teflubenzuron, Tefluthrin, Temephos, Terbam, Terbufos, Tetrachlorvinphos, Thiafenox, Thiamethoxam, Thiodicarb, Thiofanox, Thiomethon, Thionazin, Thuringiensin, Tralomethrin, Transfluthrin, Triarathen, Triazophos, Triazuron, Trichlorfon, Triflumuron, Trimethacarb,
Vamidothion, XMC, Xylylcarb, Zetamethrin.

Als Beispiele für Herbizide seien genannt:

Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfopmethyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, 4-Amino-N-(1,1-dimetylethyl)-4,5-dihydro-3-(1-methylethyl)-5-oxo-1H-1,2,4-triazole-1-carboxamid, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Als Beispiele für Pflanzenwuchsregulatoren seien Chlorcholinchlorid und Ethephon genannt.

Als Beispiele für Pflanzennährstoffe seien übliche anorganische oder organische Dünger zur Versorgung von Pflanzen mit Makro- und/oder Mikronährstoffen genannt.

Als Beispiele für Repellents seien Diethyltolylamid, Ethylhexandiol und Butopyronoxyl genannt.

Als besonders bevorzugte Beispiele für Fungizide erwähnt seien die Wirkstoffe der Formeln 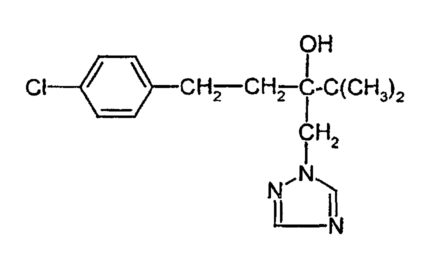 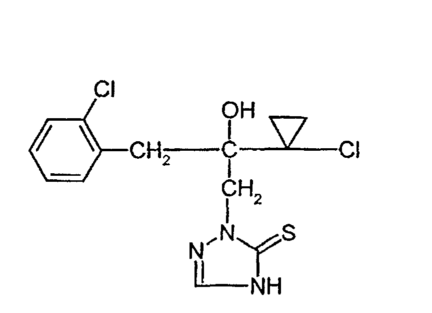 und 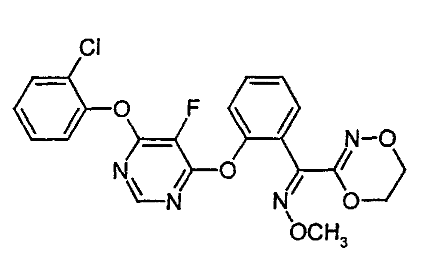 Weiterhin enthalten die erfindungsgemäßen Formulierungen 2-Ethyl-hexanol-alkoxylat der Formel (I). In dieser Formel geben die Zahlen 8 und 6 Durchschnittswerte an. Daher handelt es sich bei dem 2-Ethyl-hexanol-alkoxylat der Formel (I) um ein Substanzgemisch mit vorzugsweise 8 Propylenoxid- und 6 Ethylenoxid-Einheiten.

Das 2-Ethyl-hexanol-alkoxylat der Formel (I) ist bereits bekannt (vgl. EP-A 0 681 865).

Als Zusatzstoffe, die in den erfindungsgemäßen Formulierungen enthalten sein können, kommen alle üblichen Formulierhilfsmittel in Frage, wie z.B. organische Solventien, Emulgatoren, Dispergiermittel, Konservierungsmittel, Farbstoffe, Füllstoffe und auch Wasser.

Als organische Solventien kommen dabei alle üblichen organischen Lösungsmittel in Betracht, welche die eingesetzten agrochemischen Wirkstoffe gut lösen. Vorzugsweise genannt seien aliphatische und aromatische, gegebenenfalls halogenierte Kohlenwasserstoffe, wie Toluol, Xylol, Solvesso®, Mineralöle, wie Testbenzin, Petroleum, Alkylbenzole und Spindelöl, weiterhin Tetrachlormethan, Chloroform, Methylenchlorid und Dichlormethan, außerdem Ester, wie Ethylacetat, ferner Lactone, wie Butyrolacton, außerdem Lactame, wie N-Methylpyrrolidon, N-Octylpyrrolidon und N-Methylcaprolactam, und auch Alkancarbonsäureamide, wie Decancarbonsäure-dimethylamid und Octancarbonsäure-dimethylamid, sowie Dimethylformamid.

Als Emulgatoren kommen übliche, in Formulierungen von agrochemischen Wirkstoffen vorhandene oberflächenaktive Substanzen in Frage. Beispielhaft genannt seien ethoxylierte Nonylphenole, Polyethylenglykolether von linearen Alkoholen, Umsetzungsprodukte von Alkylphenolen mit Ethylenoxid und/oder Propylenoxid, weiterhin Fettsäureester, Alkylsulfonate, Alkylsulfate, Arylsulfate, ethoxylierte Arylalkylphenole, wie zum Beispiel Tristyryl-phenol-ethoxylat mit durchschnittlich 16 Ethylenoxid-Einheiten pro Molekül, weiterhin ethoxylierte und propoxylierte Arylalkylphenole sowie sulfatierte oder phosphatierte Arylalkylphenol-ethoxylate bzw. -ethoxy- und -propoxylate.

Als Dispergiermittel kommen alle üblicherweise in Pflanzenschutzmitteln für diesen Zweck eingesetzten Substanzen in Betracht. Vorzugsweise genannt seien natürliche und synthetische, wasserlösliche Polymere, wie Gelatine, Stärke und Cellulosederivate, insbesondere Celluloseester und Celluloseether, ferner Polyvinylalkohol, Polyvinylpyrrolidon, Polyacrylsäure, Polymethacrylsäure und Co-Polymerisate aus (Meth)acrylsäure und (Meth)acrylsäureestern, und außerdem auch mit Alkalimetallhydroxid neutralisierte Co-Polymerisate aus Methacrylsäure und Methacrylsäureester.

Als Konservierungsmittel kommen alle üblicherweise für diesen Zweck in Pflanzenbehandlungsmitteln vorhandenen Substanzen in Betracht. Als Beispiele genannt seien Preventol® und Proxel®.

Als Farbstoffe kommen alle für die Herstellung von Pflanzenschutzmitteln üblichen anorganischen oder organischen Farbstoffe in Frage. Beispielhaft genannt seien Titandioxid, Farbruß, Zinkoxid und Blaupigmente.

Als Füllstoffe kommen alle üblicherweise für diesen Zweck in Pflanzenschutzmitteln eingesetzten Substanzen in Betracht. Vorzugsweise genannt seien anorganische Partikel, wie Carbonate, Silikate und Oxide mit einer mittleren Teilchengröße von 0,005 bis 5 µm, besonders bevorzugt von 0,02 bis 2 µm. Beispielhaft erwähnt seien Siliziumdioxid, sogenannte hochdisperse Kieselsäure, Kieselgele, sowie natürliche und synthetische Silikate und Alumosilikate.

Der Gehalt an den einzelnen Komponenten kann in den erfindungsgemäßen Formulierungen innerhalb eines größeren Bereiches variiert werden. So liegen die Konzentrationen
- an agrochemischen Wirkstoffen im allgemeinen zwischen 1 und 90 Gew.-%, vorzugsweise zwischen 5 und 30 Gew.-%
- an 2-Ethyl-hexanol-alkoxylat der Formel (I) im allgemeinen zwischen 1 und 90 Gew.-%, vorzugsweise zwischen 10 und 50 Gew.-% und
- an Zusatzstoffen im allgemeinen zwischen 0 und 98 Gew.%, vorzugsweise zwischen 20 und 85 Gew.-%.

Die Herstellung der erfindungsgemäßen agrochemischen Formulierungen erfolgt in der Weise, daß man die Komponenten in den jeweils gewünschten Verhältnissen miteinander vermischt. Handelt es sich bei dem agrochemischen Wirkstoff um eine Festsubstanz, so setzt man diesen im allgemeinen entweder in fein gemahlener Form oder in Form einer Lösung oder Suspension in einem organischen Solvens ein. Ist der agrochemische Wirkstoff flüssig, so erübrigt sich häufig die Verwendung eines organischen Lösungsmittels. Es ist außerdem möglich, einen festen agrochemischen Wirkstoff in Form einer Schmelze einzusetzen.

Die Temperaturen können bei der Durchführung des erfindungsgemäßen Verfahrens in einem bestimmten Bereich variiert werden. Man arbeitet im allgemeinen bei Temperaturen zwischen 0°C und 80°C, vorzugsweise zwischen 10°C und 60°C.

Bei der Durchführung des erfindungsgemäßen Verfahrens geht man im allgemeinen so vor, daß man 2-Ethylhexanol-alkoxylat der Formel (I) mit einem oder mehreren agrochemischen Wirkstoffen sowie gegebenenfalls mit Zusatzstoffen unter Rühren intensiv vermischt. Die Reihenfolge, in der die Komponenten miteinander vermischt werden, ist beliebig. In einer bevorzugten Variante des erfindungsgemäßen Verfahrens geht man jedoch so vor, daß man 2-Ethyl-hexanol-alkoxylat der Formel (I) mit einem oder mehreren agrochemischen Wirkstoffen sowie mit weiteren Zusatzstoffen vermischt und die so entstehende Vormischung in Wasser dispergiert, so daß man Emulsionen, Suspensionen oder Lösungen erhält.

Zur Durchführung des erfindungsgemäßen Verfahrens kommen übliche Geräte in Betracht, die zur Herstellung von agrochemischen Formulierungen eingesetzt werden.

Die erfindungsgemäßen agrochemischen Formulierungen können in den für Flüssigpräparate üblichen Zubereitungsformen entweder als solche oder nach vorherigem Verdünnen mit Wasser ausgebracht werden, also z.B. als Emulsionen, Suspensionen oder Lösungen. Die Anwendung erfolgt dabei nach üblichen Methoden, also z.B. durch Verspritzen, Gießen oder Injizieren.

Die Aufwandmenge an den erfindungsgemäßen agrochemischen Formulierungen kann innerhalb eines größeren Bereiches variiert werden. Sie richtet sich nach den jeweiligen agrochemischen Wirkstoffen und nach deren Gehalt in den Formulierungen.

Mit Hilfe der erfindungsgemäßen Formulierungen lassen sich agrochemische Wirkstoffe in besonders vorteilhafter Weise auf Pflanzen und/oder deren Lebensraum ausbringen. Dabei wird eine unerwünschte Schaumbildung sowohl beim Verdünnen der Konzentrate mit Wasser als auch beim Verspritzen weitestgehend vermieden. Außerdem ist die Kristallisationsneigung fester Wirkstoffe herabgesetzt und die biologische Wirksamkeit der aktiven Komponenten wird im Vergleich zu herkömmlichen Formulierungen gesteigert.

Die Erfindung wird durch die nachfolgenden Beispiele veranschaulicht.

### Herstellungsbeispiele

### Beispiel 1

Zur Herstellung einer erfindungsgemäßen Formulierung werden 10 g an Wirkstoff der Formel 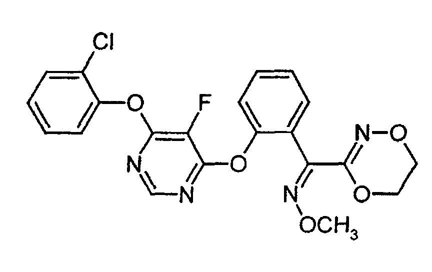 zunächst mit
40 g Butyrolacton und dann mit
50 g 2-Ethyl-hexanol-alkoxylat der Formel (I)
bei Raumtemperatur unter Rühren vermischt. Nach beendeter Zugabe wird noch 30 Minuten bei Raumtemperatur gerührt. Man erhält auf diese Weise eine homogene Lösung.

### Beispiel 2

Zur Herstellung einer erfindungsgemäßen Formulierung werden 10 g an Wirkstoff der Formel 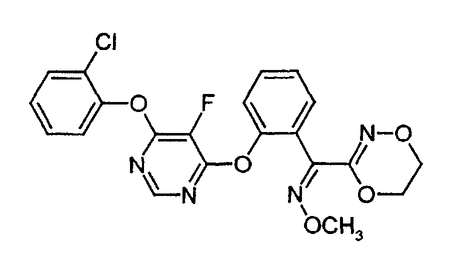 zunächst mit
35 g Butyrolacton und dann mit
35 g 2-Ethyl-hexanol-alkoxylat der Formel (I) und
20 g Tristyryl-phenol-ethoxylat mit durchschnittlich 16 Ethylenoxid-Einheiten pro Molekül
bei Raumtemperatur unter Rühren vermischt. Nach beendeter Zugabe wird noch 30 Minuten bei Raumtemperatur gerührt. Man erhält auf diese Weise eine homogene Lösung.

### Vergleichsbeispiel I

Zur Herstellung einer herkömlichen Formulierung werden 10 g an Wirkstoff der Formel 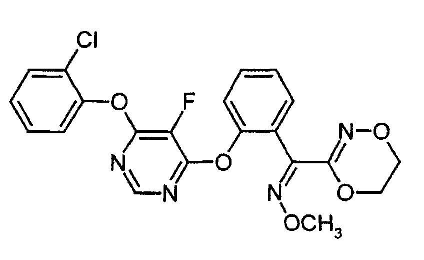 zunächst mit
70 g Butyrolacton und dann mit
20 g Tristyryl-phenol-ethoxylat mit durchschnittlich 16 Ethylenoxid-Einheiten pro Molekül
bei Raumtemperatur unter Rühren vermischt. Nach beendeter Zugabe wird noch 30 Minuten bei Raumtemperatur gerührt. Man erhält auf diese Weise eine homogene Lösung.

### Verwendungsbeispiele

### Beispiel A

### Erysiphe-Test (Gerste)/protektiv

Zur Herstellung einer anwendungsfertigen Wirkstoffzubereitung verdünnt man jeweils das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

1 Tag nach der Spritzung werden die Pflanzen mit Sporen von Erysiphe graminis f.sp. hordei bestäubt.

Die Pflanzen werden in einem Gewächshaus bei einer Temperatur von ca. 18°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Formulierungen, Aufwandmengen an Wirkstoff und Versuchsergebnisse gehen aus den folgenden Tabellen hervor.

**Tabelle A-1**

| **Erysiphe-Test (Gerste) / protektiv** | | |
|---|---|---|
| Formulierung gemäß Beispiel | Aufwandmenge an Wirkstoff in g/ha | Wirkungsgrad in % |
| Bekannt: | | |
| (I) | 62,5 | 76 |
| Erfindungsgemäß: | | |
| (1) | 62,5 | 90 |

**Tabelle A-2**

| **Erysiphe-Test (Gerste) / protektiv** | | |
|---|---|---|
| Formulierung gemäß Beispiel | Aufwandmenge an Wirkstoff in g/ha | Wirkungsgrad in % |
| Bekannt: | | |
| (I) | 62,5 | 72 |
| Erfindungsgemäß: | | |
| (2) | 62,5 | 89 |

### Beispiel B

### Erysiphe-Test (Weizen)/kurativ

Zur Herstellung einer anwendungsfertigen Wirkstoffzubereitung verdünnt man jeweils das Konzentrat mit Wasser auf die gewünschte Konzentration.

Zur Prüfung auf kurative Wirksamkeit werden junge Pflanzen mit Sporen von Erysiphe graminis f.sp. tritici bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 18°C und einer relativen Luftfeuchtigkeit von ca. 80 % aufgestellt, um die Entwicklung von Mehltaupusteln zu begünstigen.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Formulierungen, Aufwandmengen an Wirkstoff und Versuchsergebnisse gehen aus den folgenden Tabellen hervor.

**Tabelle B-1**

| **Erysiphe-Test (Weizen) / kurativ** | | |
|---|---|---|
| Formulierung gemäß Beispiel | Aufwandmenge an Wirkstoff in g/ha | Wirkungsgrad in % |
| Bekannt: | | |
| (I) | 62,5 | 38 |
| Erfindungsgemäß: | | |
| (1) | 62,5 | 86 |

**Tabelle B-2**

| **Erysiphe-Test (Weizen) / kurativ** | | |
|---|---|---|
| Formulierung gemäß Beispiel | Aufwandmenge an Wirkstoff in g/ha | Wirkungsgrad in % |
| Bekannt: | | |
| (I) | 62,5 | 60 |
| Erfindungsgemäß: | | |
| (2) | 62,5 250 ppm | 77 |

### Beispiel C

### Leptosphaeria nodorum-Test (Weizen)/kurativ

Zur Herstellung einer anwendungsfertigen Wirkstoffzubereitung verdünnt man jeweils das Konzentrat mit Wasser auf die gewünschte Konzentration.

Zur Prüfung auf kurative Wirksamkeit werden junge Pflanzen mit einer KonidienSuspension von Leptosphaeria nodorum besprüht. Die Pflanzen verbleiben 48 Stunden bei 20°C und 100 % relativer Luftfeuchtigkeit in einer Inkubationskabine und werden dann mit der Wirkstoffzubereitung in der angegebenen Aufwandmenge besprüht.

Die Pflanzen werden im Gewächshaus bei einer Temperatur von ca. 22°C und einer relativen Luftfeuchtigkeit von ca. 80 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Formulierungen, Aufwandmengen an Wirkstoff und Versuchsergebnisse gehen aus der folgenden Tabelle hervor.

**Tabelle C**

| **Leptosphaeria nodorum-Test (Weizen) / kurativ** | | |
|---|---|---|
| Formulierung gemäß Beispiel | Aufwandmenge an Wirkstoff in g/ha | Wirkungsgrad in % |
| Bekannt: | | |
| (I) | 62,5 | 87 |
| Erfindungsgemäß: | | |
| (1) | 62,5 | 100 |

### Beispiel D

### Pyrenophora teres-Test (Gerste)/kurativ

Zur Herstellung einer anwendungsfertigen Wirkstoffzubereitung verdünnt man jeweils das Konzentrat mit Wasser auf die gewünschte Konzentration.

Zur Prüfung auf kurative Wirksamkeit werden junge Pflanzen mit einer KonidienSuspension von Pyrenophora teres besprüht. Die Pflanzen verbleiben 48 Stunden bei 20°C und 100 % relativer Luftfeuchtigkeit in einer Inkubationskabine. Anschließend werden die Pflanzen mit der Wirkstoffzubereitung in der angegebenen Aufwandmenge besprüht.

Die Pflanzen werden in einem Gewächshaus bei einer Temperatur von ca. 20°C und relativen Luftfeuchtigkeit von ca. 80 % aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Formulierungen, Aufwandmengen an Wirkstoff und Versuchsergebnisse gehen aus der folgenden Tabelle hervor.

**Tabelle D**

| **Pyrenophora teres-Test (Gerste)/kurativ** | | |
|---|---|---|
| Formulierung gemäß Beispiel | Aufwandmenge an Wirkstoff in g/ha | Wirkungsgrad in % |
| Bekannt: | | |
| (I) | 62,5 | 54 |
| Erfindungsgemäß: | | |
| (1) | 62,5 | 73 |

## Patentansprüche

1. Agrochemische Formulierungen, bestehend aus
a) mindestens einem agrochemischen Wirkstoff,
b) 2-Ethyl-hexanol-alkoxylat der Formel 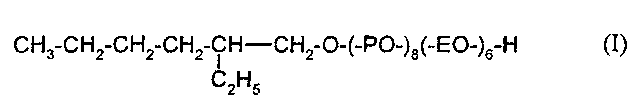 worin
P für 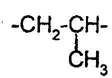 steht,
E für -CH₂-CH₂- steht und
die Zahlen 8 und 6 Durchschnittswerte darstellen,
und
c) gegebenenfalls Zusatzstoffen.

2. Agrochemische Formulierungen gemäß Anspruch 1, **dadurch gekennzeichnet, daß** als agrochemischer Wirkstoff die Verbindung der Formel 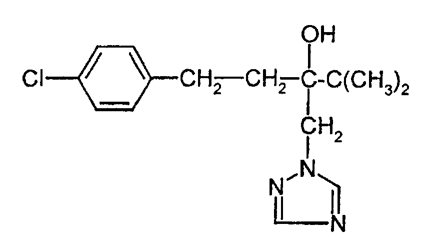 enthalten ist.

3. Agrochemische Formulierungen gemäß Anspruch 1, **dadurch gekennzeichnet, daß** als agrochemischer Wirkstoff die Verbindung der Formel 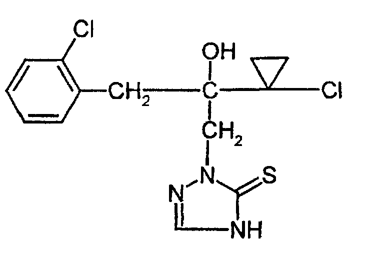 enthalten ist.

4. Agrochemische Formulierungen gemäß Anspruch 1, **dadurch gekennzeichnet, daß** als agrochemischer Wirkstoff die Verbindung der Formel 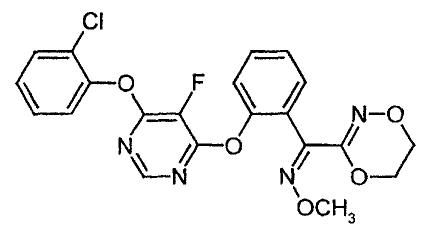 enthalten ist.

5. Verfahren zur Herstellung von agrochemischen Formulierungen gemäß Anspruch 1, **dadurch gekennzeichnet, daß** man
- mindestens einen agrochemischen Wirkstoff
- mit 2-Ethyl-hexanol-alkoxylat der Formel (I) sowie
- gegebenenfalls mit Zusatzstoffen
vermischt.

6. Verwendung von agrochemischen Formulierungen gemäß Anspruch 1 zur Applikation der enthaltenen agrochemischen Wirkstoffe auf Pflanzen und/oder deren Lebensraum.

## Claims

1. Agrochemical formulations comprising
a) at least one agrochemically active compound,
b) 2-ethyl-hexanol alkoxylate of the formula 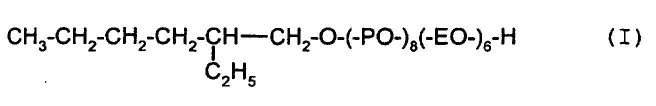 in which
P represents 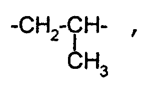
E represents -CH₂-CH₂- and
the numbers 8 and 6 are average values
and
c) optionally additives

2. Agrochemical formulations according to Claim 1, **characterized in that** they contain, as agrochemically active compound, the compound of the formula

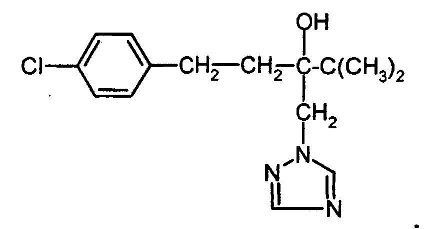

3. Agrochemical formulations according to Claim 1, **characterized in that** they contain, as agrochemically active compound, the compound of the formula

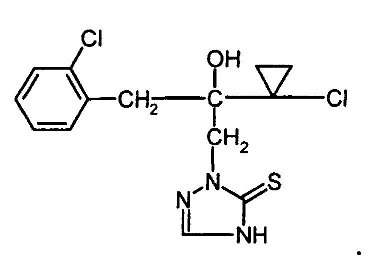

4. Agrochemical formulations according to Claim 1, **characterized in that** they contain, as agrochemically active compound, the compound of the formula

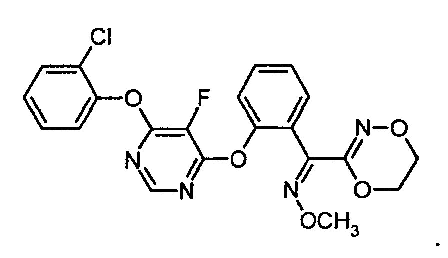

5. Process for preparing agrochemical formulations according to Claim 1, **characterized in that**
- at least one agrochemically active compound
- is mixed with 2-ethyl-hexanol alkoxylate of the formula (I) and
- optionally with additives.

6. Use of agrochemical formulations according to Claim 1 for applying the agrochemically active compounds they comprise to plants and/or their habitat.

## Revendications

1. Formulations agrochimiques, consistant en :
a) au moins un agent actif agrochimique ;
b) un alcoxylate de 2-éthylhexanol de formule : 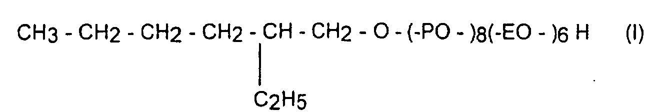 où :
P représente -CH₂-CH(CH₃)- ;
E représente -CH₂-CH₂-, et
les nombres 6 et 8 sont des valeurs moyennes,
et
c) le cas échéant, des additifs.

2. Formulations agrochimiques suivant la revendication 1, **caractérisées en ce que** le composé de formule : 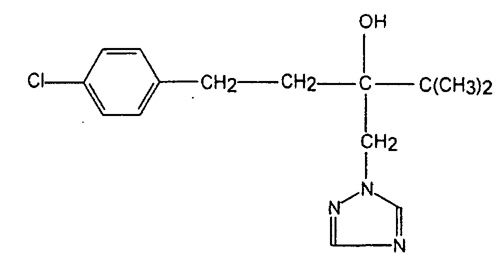 est présent en tant qu'agent actif agrochimique.

3. Formulations agrochimiques suivant la revendication 1, **caractérisées en ce que** le composé de formule : 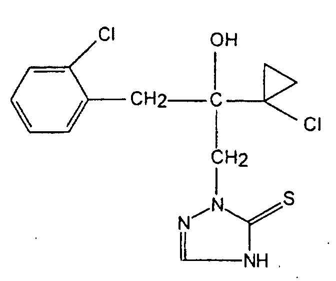 est présent en tant qu'agent actif agrochimique.

4. Formulations agrochimiques suivant la revendication 1, **caractérisées en ce que** le composé de formule : 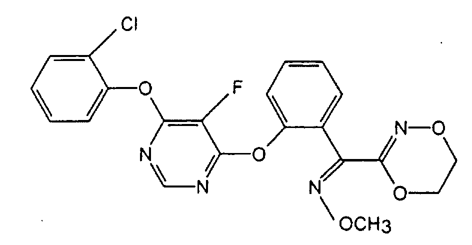 est présent en tant qu'agent actif agrochimique.

5. Procédé de préparation de formulations agrochimiques suivant la revendication 1, **caractérisé en ce que** l'on mélange :
- au moins un agent actif agrochimique
- avec un alcoxylate de 2-éthylhexanol de formule (I), ainsi que
- le cas échéant, des additifs.

6. Utilisation des formulations agrochimiques suivant la revendication 1, pour l'application des agents actifs agrochimiques qu'elles contiennent, sur des végétaux et/ou leur biotope.
